# FASCICULE DE BREVET EUROPEEN

(11) **EP 1 965 800 B1**
(45) Date de publication et mention de la délivrance du brevet: **02.11.2011**
(21) Numéro de dépôt: 06847186.1
(22) Date de dépôt: 20.12.2006
(51) Int. Cl.: A61K 31/445, A61P 13/00, A61P 1/12, A61P 43/00

(54) **DERIVES DE PIPERIDINE POUR LEUR UTILISATION DANS LE TRAITEMENT DES INCONTINENCES**
PIPERIDINE-DERIVATE ZUR BEHANDLUNG VON INKONTINENZEN
PIPERIDINE DERIVATIVES FOR USE TO TREAT INCONTINENCES

(30) Priorité: 22.12.2005 FR 0513163
(43) Date de publication de la demande: 10.09.2008
(73) Titulaire: UROSPHERE SAS, 31682 Labège Cedex (FR)
(72) Inventeur: LLUEL, Philippe, F-31520 Ramonville Saint Agne (FR); PALEA, Stefano, F-31400 Toulouse (FR)
(74) Mandataire: Becker, Philippe
(86) Numéro de dépôt international: PCT/FR2006/051396
(87) Numéro de publication internationale: WO 2007/074291

(56) Documents cités:
- EP-A- 0 077 427
- EP-A2- 1 199 069
- DOI T ET AL: "EFFECTS OF TAK-637, A TACHYKININ RECEPTOR ANTAGONIST, ON LOWER URINARY TRACT FUNCTION IN THE GUINEA PIG" EUROPEAN JOURNAL OF PHARMACOLOGY, AMSTERDAM, NL, vol. 383, 1999, pages 297-303, XP002950305 ISSN: 0014-2999
- RAO S.S.: "Diagnosis and management of fecal incontinence" AM. J. GASTROENTEROLOGY, vol. 99, 2004, pages 1583-1604, XP002436073 cité dans la demande

## Description

La présente invention appartient aux domaines de la chimie pharmaceutique et des traitements pharmacologiques. Elle concerne plus particulièrement des compositions et méthodes pour le traitement des incontinences, des troubles mictionnels associés aux dysfonctionnements du bas appareil urinaire, et/ou des troubles sphinctériens urétro-vésicaux et anaux. L'invention est utilisable notamment pour le traitement de la pollakiurie, l'impétuosité ou l'urgence urinaire, la nocturie ou l'énurésie, l'urgence fécale et le suintement fécal, et est applicable chez tous mammifères, en particulier chez l'être humain, à titre préventif ou curatif.

Le bas appareil urinaire dédié au stockage de l'urine (continence) et à son élimination (miction) se compose des uretères, de la vessie, des sphincters urinaires et de l'urètre. Chez l'homme, la prostate lui est associée du fait des répercussions mictionnelles fréquentes que peuvent avoir les infections, les inflammations ou les hyperplasies prostatiques.

L'incontinence urinaire se définit par une perte involontaire d'urine et résulte d'une déficience du contrôle de la vessie et/ou des muscles des sphincters urinaires. En dehors de la miction où ils sont volontairement relâchés, les sphincters urinaires sont suffisamment contractés pour contenir la pression exercée par le muscle vésical. L'incontinence urinaire survient lorsque la pression vésicale est trop forte ou lorsque la contraction des sphincters urinaires est trop faible pour contenir une pression intra vésicale normale.

L'incontinence urinaire existe sous différentes formes : l'incontinence d'effort, l'incontinence d'urgence, l'incontinence mixte, l'incontinence par regorgement et l'incontinence fonctionnelle.

L'incontinence urinaire d'effort se manifeste par une perte d'urine lorsqu'une pression est exercée au niveau de l'abdomen (exercice physique, accès de toux ou éclat de rire). Cette forme d'incontinence, liée à un dysfonctionnement des sphincters urinaires et de l'urètre, se rencontre principalement chez la femme.

L'incontinence urinaire d'urgence se manifeste par une incapacité à contenir une forte envie d'uriner et résulte d'une diminution de la capacité de remplissage vésical (manque de compliance) ou d'une contraction anormale du détrusor pendant la phase de remplissage de la vessie. Elle touche essentiellement les sujets âgés. Selon la cause neurologique ou non du dysfonctionnement du muscle vésical, on distingue deux pathologies : l'hyperréflexie du détrusor et l'instabilité du détrusor. L'hyperréflexie du détrusor se manifeste lors d'atteintes neurologiques, en particulier la sclérose en plaques, les lésions de la moelle épinière, les neuropathies périphériques ou les tumeurs cérébrales. A l'opposé, l'instabilité du détrusor peut avoir des causes diverses, infectieuses, inflammatoires ou hormonales et peut également être induite par une hypertrophie prostatique. 75 % des cas d'hypertrophie bénigne de la prostate sont, en effet, accompagnés d'une hyperactivité vésicale (Scrip Reports, Sept. 2000).

Dans les incontinences urinaires mixtes, les symptomatologies des incontinences d'effort et d'urgence sont associées.

L'incontinence urinaire par regorgement survient par un trop plein de la vessie suite à une rétention d'urine. Cette forme d'incontinence peut survenir lorsque la vidange de la vessie est contrariée par une obstruction, comme dans l'hypertrophie prostatique, ou lorsque cette vidange est incomplète par manque de contractilité du muscle vésical. Cette forme d'incontinence se rencontre en particulier chez les sujets diabétiques en raison des neuropathies périphériques qu'ils développent (Scrip Reports, Sept. 2000).

L'incontinence urinaire fonctionnelle correspond à une incapacité à retenir son urine pour une cause indépendante de toute perturbation neuro-urologique ou de tout dysfonctionnement du tractus urinaire bas. 75% des patients souffrant d'atteintes neurologiques graves telles la maladie d'Alzheimer, la maladie de Parkinson, ou les séquelles d'accidents vasculaires cérébraux sont touchés par une incontinence urinaire (Scrip Reports, Sept. 2000).

L'incontinence urinaire et les troubles mictionnels touchent plusieurs centaines de millions de personnes dans le monde (Scrip Reports, Sept. 2000). S'ils ne menacent pas le pronostic vital, ces désordres altèrent considérablement la qualité de vie des patients. Ils peuvent devenir un véritable obstacle dans le cadre de la vie socioprofessionnelle et avoir d'importantes répercussions physiologiques et psychologiques.

Outre les incontinences urinaires, d'autres troubles ou désordres mictionnels sont associés à des dysfonctionnements de composants du bas appareil urinaire, tels que la pollakiurie (envie fréquente d'uriner avec une miction très peu abondante), l'impétuosité ou l'urgence urinaire (besoin urgent d'uriner), la nocturie (besoin fréquent d'uriner pendant la nuit) ou l'énurésie (miction involontaire ou incapacité à contenir ses urines). Ces désordres mictionnels se manifestent dans de nombreuses pathologies, notamment et non exclusivement, l'instabilité vésicale, l'hyperactivité vésicale, la cystite, la cystite interstitielle et les maladies prostatiques (hypertrophie bénigne de la prostate, hyperplasie prostatique, prostatite et prostadynie). Ils se rencontrent également chez des patients souffrant de diabète, de traumatismes de la moelle épinière ou d'atteintes cérébrales (maladie d'Alzheimer, maladie de Parkinson, tumeurs ou accidents vasculaires) et peuvent même avoir une origine iatrogène (Scrip Reports, Sept. 2000).

Les traitements disponibles aujourd'hui sont peu nombreux. En dehors des anticholinergiques prescrits dans l'incontinence d'urgence, des α-bloquants utilisés pour les troubles urinaires des patients atteints d'hypertrophie bénigne de la prostate et d'un inhibiteur mixte de la recapture de la norépinéphrine et de la sérotonine, la duloxétine, récemment enregistré en Europe dans l'incontinence d'effort, l'arsenal thérapeutique se résume à un détournement d'usage d'oestrogènes, d'anti-dépresseurs tricycliques et d'agonistes α₁-adrénergiques. (EP 1 199 069; Doi et coll., 1999, EJP (383), 297- 303). Peu efficaces et souvent mal tolérés, ces traitements conduisent fréquemment à une mauvaise compliance des patients voire à des arrêts de traitement (Scrip Reports, Sept. 2000).

L'incontinence fécale (IF) est définie par une incapacité à contenir les selles (solides ou liquides) après l'apprentissage de la propreté (Cooper Z.R. et coll., 2000). L'IF se distingue de l'incontinence anale (IA) par la nature des pertes anales. Alors que l'IF concerne uniquement les pertes de selles (solides ou liquides), l'IA s'étend à la perte de gaz (Macmillan A.K. et coll., 2004). On distingue «l'incontinence fécale vraie», liée de la perte du contrôle du sphincter anal, de l'incontinence fécale fonctionnelle (IFF) correspondant à une perte récurrente de selles en l'absence d'altération neurologique ou structurale du contrôle du sphincter anal (Whitehead W.E. et coll., 2006). L'IFF regroupe des pathologies pouvant aussi bien avoir une origine anale (hémorroïdes, fistule anale, prolapsus de la muqueuse fécale) qu'intestinale (abus de laxatifs, pathologies inflammatoires ou parasitaires de l'intestin). Sur le plan clinique, il existe trois sous-types d'IF : l'incontinence fécale passive (perte de selles involontaire et sans perceptions), l'incontinence fécale d'urgence ou l'urgence fécale (perte fécale en dépit de tentative à contenir le bol fécal) et le suintement fécal (fuite fécale, alors que la défécation est normale) (Rao S., 2004). L'IF passive se manifeste lors d'un dysfonctionnement du sphincter anal interne ou lors d'une obstruction du rectum par des selles (survenue d'un «débordement» de selles liquides autour de l'obstacle causé par la constipation) (Kamm M.A., 1998). L'incontinence fécale d'urgence peut résulter d'une atteinte ou d'un dysfonctionnement du sphincter anal externe, mais peut également être consécutive à une augmentation de la pression intestinale, alors que le sphincter est intact (Ex : diarrhées d'origines diverses, syndrome du colon irritable) (Engel A.F. et coll., 1995).

L'incontinence fécale est un désordre complexe et multifactoriel, dont les origines peuvent être très diverses. Les atteintes du sphincter (de la faiblesse à la rupture), les neuropathies du nerf honteux, les altérations sensitives anorectales, les altérations de la compliance rectale, la défécation incomplète sont autant de causes possibles à la survenue d'une IF ; ces atteintes pouvant elles-mêmes avoir des origines différentes (anatomiques, locales ou systémiques). Il est, par ailleurs, fréquent qu'une IF ait des causes multiples (Bharucha A.E., 2003 et Cooper Z.R. et coll., 2000).

Il semble couramment admis que la prévalence de l'IF serait de l'ordre de 2% pour la population générale, d'environ 7% pour les personnes autonomes de plus de 65 ans et de 25-33% pour les personnes âgées institutionnalisées ou hospitalisées (avec, pour cette population, une association très fréquente à une incontinence urinaire) (Kamm M.A., 1998).

Des études récentes ont montré une association fréquente entre incontinence urinaire et incontinence fécale. Aux Etats-Unis, dans une population mixte de plus de 50 ans, la prévalence de la double incontinence est de 5.9 % (hommes) et 9.4% (femmes) (Roberts R.O. et coll., 1999). Des prévalences similaires (8.4% et 8.7%) ont été rapportées pour les femmes en Europe (Griffiths A.N. et coll, 2006 et Lacima G. et coll., 2002). L'association très fréquente des incontinences urinaire et fécale pourrait s'expliquer par les similitudes existant dans le fonctionnement du sphincter urétral et du sphincter anal (Leroi et Le Normand, 2005). La mise en évidence, chez l'animal, de réflexes croisés entre la vessie, l'urètre, le complexe ano-rectal et le plancher pelvien a, également, été mise en avant pour expliquer, au moins en partie, la co-mobidité de ces deux incontinences (Kapoor et coll., 2005).

A côté des couches de protection, des mesures diététiques, des techniques de rééducation (biofeedback, rééducation pelvienne), de divers dispositifs médicaux et de la chirurgie (réparation du sphincter, transposition musculaire, sphincter artificiel, stimulation du nerf sacré, colostomie, etc.), les traitements pharmacologiques proposés ou étudiés dans le traitement de l'IF sont, à ce jour, peu nombreux. Dans la plupart des cas, il s'agit de traitements non spécifiques destinés à agir sur la cause suspectée de l'incontinence, i.e. la diarrhée ou la constipation (Rao S., 2004 ; Bharucha A.E., 2003).

Face au nombre considérable de patients atteints par une incontinence urinaire, un désordre mictionnel, une incontinence fécale ou anale et/ou des troubles sphinctériens urétro-vésicaux et anaux, et à l'inadéquation des traitements disponibles, il existe un besoin évident de traitements efficaces dénués d'effets secondaires.

La présente invention propose maintenant de nouvelles méthodes efficaces de traitement de ces pathologies. Il a été observé, de façon inattendue, que certains dérivés de la pipéridine, dont la préparation et l'activité antidépressive sont révélées dans le brevet N° EP77427, ont un effet pharmacologique sur la vessie et le système musculo-sphinctérien urétral et anal dont les dysfonctionnements sont impliqués dans les incontinences urinaire, fécale ou anale. En particulier, les exemples dévoilés dans la présente demande montrent que, de façon inattendue, ces composés, administrés par voie intraveineuse, augmentent la capacité vésicale et l'activité électromyographique du sphincter anal strié ; cette dernière étant notamment reconnue dans la littérature pour être représentative de celle du sphincter urétral (Thor et Muhlhauser, 1999 et Wenzel et coll., 2006). Par ailleurs, *in vitro,* ces composés inhibent, de façon dépendante de la concentration, la réponse contractile de la vessie humaine stimulée électriquement et inhibent la potentialisation par la sérotonine de la réponse neurogénique de la vessie de rat (réponse contractile à la stimulation électrique). Un objet de l'invention réside donc dans l'utilisation, pour la fabrication d'un médicament pour le traitement des incontinences, des troubles mictionnels associés aux dysfonctionnements du bas appareil urinaire et/ou des troubles sphinctériens urétro-vésicaux et anaux, d'un composé de formule (I) 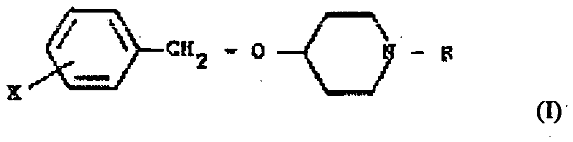 dans laquelle
R représente un atome d'hydrogène ou un radical (C1-4)alkyle, hydroxy-(C1-4)alkyle, (C1-4)alcoxycarbonyle ou benzyle, lesdits radicaux étant éventuellement substitués par un ou plusieurs substituants choisi(s) de préférence parmi un atome d'halogène et un radical (C1-4)alcoxy, phénéthyle ou phényl-3 propyle,
X représente un ou plusieurs atomes d'hydrogène ou d'halogène ou radicaux choisis parmi (C1-4)alkyle, (C1-4)alcoxy, trifluorométhyle et méthylènedioxy, ou bien X forme avec le noyau phényle un radical naphtyle, ainsi que ses sels et hydrates acceptables sur le plan pharmaceutique.

Dans le contexte de l'invention, le terme incontinence désigne les incontinences urinaires, fécales ou anales.

Dans le contexte de l'invention, le terme (C1-4)alkyle désigne plus préférentiellement des groupes méthyle, éthyle, propyle ou isopropyle, et butyle. Parmi les groupes hydroxy-(C1-4)alkyle, on peut citer à titre plus spécifique les groupes méthoxy et éthoxy. Parmi les radicaux (C1-4)alcoxycarbonyle, on peut mentionner notamment C(O)OCH₃ et C(O)OCH₂CH₃.

Dans un mode de mise en oeuvre préféré, R et X ne représentent pas simultanément des atomes d'hydrogène. Des exemples de tels composés sont notamment les composés 1 à 49 décrits dans le brevet EP 077 427.

Des composés particuliers sont ceux dans lesquels X représente un ou plusieurs atomes de chlore, ou forme avec le noyau phényle un radical naphtyle, ou encore représente trois radicaux méthoxy. Des composés tout particulièrement préférés sont ceux dans lesquels X forme avec le noyau phényle un radical naphtyle.

Un autre groupe de composés préférés est celui dans lesquels R est H.

Un exemple tout particulièrement préféré de composé est la 4-[(2-naphtalényl)-méthoxy]-pipéridine, de formule suivante (composé **A**) :

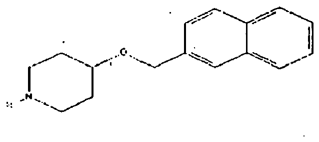

Parmi les sels d'addition acceptables sur le plan pharmaceutique, on peut citer en particulier les sels d'acides, tels que notamment le benzoate, le mandélate, le chlorhydrate, le citrate et le fumarate.

Les composés tels que mentionnés ci-dessus peuvent être produits par différentes techniques de synthèse, connues en soi de l'homme du métier. Dans ce contexte, des procédés de synthèse, qui peuvent être mis en oeuvre pour les besoins de la présente demande, sont décrits en détails dans le brevet EP 077 427.

Dans un mode particulier de l'invention, le composé de formule I utilisé présente une propriété d'inhibition de la recapture de la sérotonine. Plus préférentiellement encore, il s'agit d'un inhibiteur sélectif de la recapture de la sérotonine. Au sens de l'invention, on entend par le terme inhibiteur sélectif de la recapture de la sérotonine un composé qui, aux doses utilisées, ne présente pas d'effet substantiel sur la recapture de la norépinéphrine ou de la dopamine. Avantageusement, un inhibiteur de la recapture de la sérotonine est sélectif lorsque le rapport d'inhibition IC50_{NE/HT} et/ou IC50_{DA/HT} est supérieur à 30, 40, 50, 60 70 ou 80, de préférence compris entre 50 et 300.

De manière particulièrement avantageuse, le composé de formule I est en outre un agoniste, partiel ou non, des récepteurs 5-HT_{2C} et/ou un antagoniste des récepteurs 5-HT₃ et/ou des récepteurs 5-HT₄ et/ou récepteurs 5-HT₇. La présente demande montre en effet que des composés présentant un profil d'inhibiteur sélectif de la recapture de la sérotonine combiné à une activité agoniste, partiel ou non, des récepteurs 5-HT_{2C} et/ou antagoniste des récepteurs 5-HT₄ et/ou 5-HT₇ produisent un effet particulièrement avantageux sur la pression urétrale et sur la réponse contractile de la vessie et limitent les risques de vomissements du fait de leur activité antagoniste sur les récepteurs 5-HT₃. Le terme agoniste d'un récepteur désigne tout composé capable de lier ce récepteur et de mimer la réponse induite par le ligand naturel de ce récepteur. Le terme antagoniste d'un récepteur désigne tout composé capable de lier ce récepteur et de bloquer la réponse induite par le ligand naturel de ce récepteur.

Des composés de formule I tout particulièrement préférés sont ceux qui présentent, en combinaison avec l'une, des ou toutes les propriétés mentionnées ci-dessus, une faible affinité pour les récepteurs dopaminergiques D₂ (c'est-à-dire inférieure d'un facteur 5, 10, 20 ou 30 au moins par rapport à l'affinité pour le transporteur de la sérotonine) et/ou pour les récepteurs adrénergiques (c'est-à-dire inférieure d'un facteur 5, 10, 20 ou 30 au moins par rapport à l'affinité pour le transporteur de la sérotonine) et/ou pour les récepteurs muscariniques (c'est-à-dire inférieure d'un facteur 5, 10, 20 ou 30 au moins par rapport à l'affinité pour le transporteur de la sérotonine). En effet, cette sélectivité permet de disposer de composés n'induisant pas de nausées ni les effets secondaires cardiovasculaires (hypertension artérielle) et anticholinergiques (xérostomie) fréquemment rencontrés avec les traitements actuellement disponibles.

Ainsi, un objet particulier de l'invention réside également dans l'utilisation, pour la fabrication d'un médicament pour le traitement des incontinences, des troubles mictionnels associés aux dysfonctionnements du bas appareil urinaire, et/ou des troubles sphinctériens urétro-vésicaux et anaux, d'un composé de formule I inhibiteur sélectif de la recapture de la sérotonine, agoniste, partiel ou non, des récepteurs 5-HT_{2C} et/ou antagoniste des récepteurs 5-HT₃ et/ou des récepteurs 5-HT₄ et/ou récepteurs 5-HT₇ et présentant une faible affinité pour les récepteurs dopaminergiques D₂, les récepteurs adrénergiques et les récepteurs muscariniques.

Un exemple spécifique d'un tel composé est la 4-[(2-naphtalényl)-méthoxy]-pipéridine. Les demandeurs ont en effet montré que ce composé présentait une nette sélectivité sur la recapture de la sérotonine (5-HT) par rapport à celle de la norépinéphrine (NE) (Ratio IC_{50 NE/HT} = 89), (Scatton, 1988) et une très faible affinité pour les récepteurs adrénergiques [α₁ (IC₅₀ = 40 µM), α₂ (IC₅₀ = 70 µM) et β (IC₅₀ = 100 µM)] et pour les récepteurs muscariniques (IC₅₀ = 99 µM). Du fait de cette sélectivité, ce composé ne devrait pas entraîner les effets secondaires cardiovasculaires (hypertension artérielle) et anticholinergiques (xérostomie) fréquemment rencontrés avec les traitements actuellement disponibles. De plus, la nette sélectivité de la 4-[(2-naphtalényl)-méthoxy]-pipéridine sur la recapture de la sérotonine par rapport à celle de la dopamine (DA) (Ratio IC_{50 DA/HT} = 188), (Scatton, 1988), son activité antagoniste sur le récepteur 5-HT₃ et ses propriétés antiémétiques chez l'animal (Angel, 1993) devraient réduire voire supprimer les vomissements qui accompagnent fréquemment le traitement par la duloxétine. En outre, des études de liaison sur des récepteurs humains clonés ont montré que la 4-[(2-naphtalényl)-méthoxy]-pipéridine présentait des affinités significatives pour les récepteurs 5-HT_{2A}, 5-HT_{2B}, 5-HT_{2C}, 5-HT₃, 5-HT_{4c} et 5-HT₇ avec des IC₅₀ comprises entre 0,25 µM et 7,2 µM, mais une faible affinité pour les récepteurs 5-HT_{1A} et 5-HT_{5A}. Il a de plus été montré pour la première fois que ce composé était un agoniste partiel pour le récepteur 5-HT_{2C}, avec une IC₅₀ de 1,5 µM, ce qui constitue un avantage particulier dans le traitement des pathologies impliquant une activité anormale de la vessie (Steers, 1989 ; Steers, 1992 ; Guarneri, 1996 ; Leysen, 1999). En outre, l'inhibition inattendue de la potentialisation par la sérotonine de la réponse neurogénique de la vessie isolée de rat stimulée électriquement, observée à 10 µM pourrait mettre en jeu une activité antagoniste sur les récepteurs 5-HT₇ (Palea, 2004), ce qui présente un intérêt dans le traitement des incontinences urinaires d'urgence et des incontinences urinaires mixtes du fait de la présence des récepteurs 5-HT₇ dans la vessie humaine (D'Agostino, 2006).

Un objet tout particulier de l'invention réside donc dans l'utilisation de la 4-[(2-naphtalényl)-méthoxy]-pipéridine, ou un sel de celle-ci, pour la préparation d'un médicament pour le traitement des incontinences, des troubles mictionnels associés aux dysfonctionnements du bas appareil urinaire, et/ou des troubles sphinctériens urétro-vésicaux et anaux.

L'invention est utilisable notamment pour le traitement des incontinences urinaires et des troubles mictionnels associés aux dysfonctionnements du bas appareil urinaire, et notamment de la pollakiurie, l'impétuosité ou l'urgence urinaire, la nocturie ou l'énurésie. Ces désordres mictionnels se manifestent dans de nombreuses pathologies, notamment l'instabilité vésicale, l'hyperactivité vésicale, la cystite, la cystite interstitielle et les maladies prostatiques (hypertrophie bénigne de la prostate, hyperplasie prostatique, prostatite et prostadynie). Ils se rencontrent également chez des patients souffrant de diabète, de traumatismes de la moelle épinière ou d'atteintes cérébrales (maladie d'Alzheimer, maladie de Parkinson, tumeurs ou accidents vasculaires) et peuvent même avoir une origine iatrogène.

Elle est également particulièrement adaptée au traitement des incontinences anales et fécales, et notamment des incontinences fécales vraies et des incontinences fécales fonctionnelles (IFF). Elle est utile aussi bien dans le traitement des incontinences fécales passives, des incontinences fécales d'urgence que du suintement fécal.

Au sens de l'invention, le terme traitement désigne aussi bien un traitement curatif que préventif. Ce terme englobe toute amélioration des symptômes de la maladie ou toute diminution des manifestations des troubles considérés, et notamment une diminution de leur fréquence, de la gêne ou de l'inconfort occasionnés, de la douleur, voire une disparition totale des troubles. D'autre part, le traitement peut être utilisé seul ou en combinaison avec d'autres principes actifs, soit de manière simultanée, soit de manière séparée ou espacée dans le temps.

La méthode de traitement décrite dans la présente invention comprend l'administration d'une dose thérapeutique efficace à un patient nécessitant ce type de traitement. Le terme dose thérapeutique efficace désigne une quantité suffisante de composé pour traiter l'incontinence ou pour obtenir une diminution, complète ou partielle, d'au moins un des symptômes sélectionnés parmi la pollakiurie, l'impétuosité urinaire, la nocturie, l'énurésie, l'urgence fécale ou le suintement fécal, par exemple.

La dose efficace, variable, est déterminée par le médecin en charge du patient. Cette dose efficace peut nécessiter un ajustement lorsque le composé est administré sous forme de sel, en particulier lorsque celui-ci a une masse moléculaire importante.

La gamme de doses efficaces est typiquement être comprise entre 0,001 et 1000 mg/jour. La dose efficace est formulée dans une préparation pharmaceutique adéquate et peut être, en fonction du besoin, contenue en totalité dans une dose journalière de cette préparation ou être administrée par fractions à différentes heures de la journée.

Selon l'invention, les composés peuvent être administrés par voie orale, buccale, sublinguale, rectale, vaginale, nasale, transcutanée, parentérale, intra-vésicale, trans-urétrale ou systémique. La voie d'administration n'est pas un élément critique de l'invention. Le composé étant absorbé au niveau du tractus digestif, il est préférentiellement administré par voie orale, pour des raisons de commodité, mais il peut, si besoin, être administré par n'importe quelle voie acceptable sur le plan pharmaceutique.

Les composés utilisés dans l'invention peuvent être administrés sous toutes les formes pharmaceutiques usuelles, telles que les comprimés enrobés ou non, les comprimés à croquer ou à sucer, les pastilles, les gélules, les capsules molles, les solutions, les suspensions aqueuses ou huileuses, les émulsions, les solutions injectables, les suppositoires, les sirops, les granules, les poudres, les patchs, les gels, les crèmes, les pommades, les sprays et les aérosols.

Ces préparations pharmaceutiques peuvent être formulées de façon à contenir la dose journalière ou une fraction de la dose journalière dans une unité de dosage qui peut être une entité solide, comme le comprimé, ou un volume adéquat d'une préparation solide, liquide ou semi solide. En fonction du besoin en terme de délai et de durée d'action, le composé peut être administré dans une formulation à libération contrôlée (retardée, prolongée, programmée, pulsatile).

L'activité du composé ne dépend pas de la composition des formulations dans lesquelles il est administré, ni de la concentration du composé dans ces formulations.

Les formulations sont préparées avec des excipients pharmaceutiquement acceptables, choisis selon les pratiques pharmaceutiques usuelles, en fonction de la forme pharmaceutique souhaitée. Dans le cas des formulations pharmaceutiques orales solides, ces excipients incluent notamment des agents liants (hydroxypropylcellulose, polyvinylpyrrolidone, amidon), des diluants inertes (lactose, carbonate de calcium, cellulose microcristalline), des lubrifiants (silice, talc, stéarate de magnésium, acide stéarique), des désintégrants (glycolate sodique d'amidon, acide alginique), des agents mouillants (laurylsulfate de sodium) et/ou des agents d'enrobage ou de pelliculage aqueux ou non aqueux.

Les préparations liquides peuvent utiliser des véhicules aqueux (eau, mélanges hydroalcooliques, sérum physiologique, tampons) ou non aqueux (propylène glycol, polyéthylène glycol, huiles, esters organiques injectables tel que l'oléate d'éthyle). Elles peuvent contenir des agents de suspension (sorbitol, méthylcellulose), des émulsifiants (gommes, lécithines), des conservateurs, des agents d'aromatisation, des colorants et/ou des édulcorants.

Les préparations topiques peuvent contenir des agents promoteurs d'absorption.

D'autres aspects et avantages de l'invention seront illustrés dans les exemples qui suivent, qui doivent être considérés comme illustratifs et non limitatifs.

### Légende des Figures

Figure 1 : Augmentation de l'activité du sphincter anal strié (A) et de la capacité vésicale (B) par le composé A chez le lapin femelle anesthésié dans des conditions d'irritation vésicale induites par perfusion locale d'acide acétique dilué.
Fleure 2 : Augmentation de la capacité vésicale par le composé A chez le rat femelle anesthésié dans des conditions d'irritation vésicale induites par perfusion locale d'acide acétique dilué.
Figure 3 : Antagonisme par le composé A de la potentialisation de la réponse neurogénique induite par la 5-HT dans la vessie isolée de rat.

### Exemples

### Exemple 1 : Effet de composés de formule (I) sur l'activité du sphincter anal strié et sur la capacité vésicale in vivo chez le lapin femelle anesthésié dans des conditions d'irritation vésicale induites par perfusion intra-vésicale d'acide acétique dilué

L'effet de la 4-[(2-naphtalényl)-méthoxy]-pipéridine (composé **A**) et de son solvant (NaCl 0,9%) sur l'activité électromyographique du sphincter anal strié (EMG-SAS), utilisé comme marqueur de l'activité du sphincter urétral et sur la capacité vésicale (CV) a été étudié *in vivo* chez le lapin femelle anesthésié dans des conditions d'irritation vésicale induites par une perfusion intra-vésicale d'acide acétique dilué (Perez Martinez et coll., 2006 et Haab et coll., 2006). L'activité du sphincter anal strié (SAS) est, en effet, reconnue dans la littérature pour être notamment représentative de celle du sphincter urétral (Thor et Muhlhauser, 1999 et Wenzel et coll., 2006). 16 lapins Néo Zélandais blancs femelles (2,5-3,5 kg), anesthésiés par une injection intramusculaire d'un mélange de kétamine (25 mg/kg) et de xylazine (10 mg/kg), ont été soumis à une cystostomie. Après laparotomie par une incision médiane, un cathéter a été introduit au niveau du dôme de la vessie et maintenu en place par une suture en bourse. Un bouchon multiperforable a été placé à l'extrémité du cathéter, puis le cathéter a été internalisé en sous cutanée et fixé au niveau abdominal par une ligature de soie. 3 jours après la cystotomie, une cystomanométrie a été réalisée sur les animaux anesthésiés à l'halothane (2-3%). Un tube en T a été connecté au niveau du bouchon multiperforable, puis relié à un capteur de pression (Letica, PanLab) et à une micro-pompe permettant la perfusion de la vessie (Razel 99, Scientific Instruments). De l'acide acétique dilué (0,5%), maintenu à température ambiante, a été perfusé dans la vessie (1,4 mL/h), afin de produire des mictions successives dans des conditions d'irritation vésicale. Le profil mictionnel a été enregistré en continu grâce à un système d'acquisition de données (PowerLab 4/25, PanLab) et les différents paramètres urodynamiques, dont la capacité vésicale (CV), ont été déterminés. Par ailleurs, deux électrodes ont été introduites au niveau du sphincter anal strié de façon à mesurer son activité électromyographique (EMG). Le signal électrique, amplifié et filtré (bande passante 1 Hz-5kHz) a été enregistré en continu, grâce au système d'acquisition de données. L'EMG-SAS a été mesurée pendant la phase de remplissage de la vessie, de façon à s'affranchir des parasites liés aux mouvements périnéaux se produisant pendant la miction.

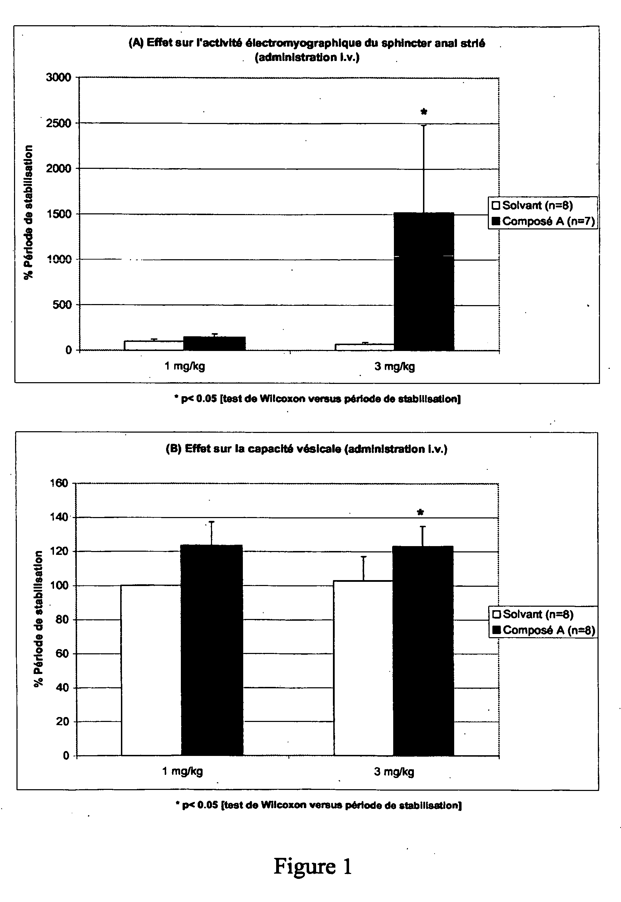

Un cathéter a été implanté au niveau de la veine de l'oreille pour permettre l'administration du composé **A** ou de son solvant (NaCl 0,9%) sous un volume d'1 mL. Les animaux ont été répartis en deux groupes recevant le composé **A** ou son solvant. Après une période de stabilisation (PS) d'au moins 60 minutes, pendant laquelle la vessie a été perfusée, afin d'induire des cycles mictionnels réguliers, deux doses consécutives (1 et 3 mg/kg) de composé **A** ou de solvant ont été administrées par voie intraveineuse, à 40 minutes d'intervalle. Pour chacune des doses testées, les effets du composé **A** et de son solvant sur l'EMG-SAS et sur la CV ont été exprimés en pourcentage de la période de stabilisation (moyenne ± erreur standard de la moyenne). Pendant la période de stabilisation, les paramètres obtenus dans les deux groupes expérimentaux (composé **A** et solvant) étaient identiques (p>0,05 ; test de Kruskal-Wallis) : pour l'EMG-SAS, 1,76 ± 1,04 (n=8) et 1,41 ± 0,59 pics d'activité/minute (n=8), respectivement pour le composé **A** et son solvant et pour la CV, 21,63 ± 4,92 (n=8) et 18,31 ± 2,90 mL (n=8), respectivement pour le composé **A** et son solvant. Alors qu'à la dose de 1 mg/kg le composé **A** n'a induit aucun d'effet significatif sur les paramètres mesurés, il a produit à la dose de 3 mg/kg une augmentation significative de l'EMG-SAS [1521,90 ± 966,61% (n=7), p=0,018 (test de Wilcoxon)] et de la CV [123,29 ± 11,62% (n=8), p=0,035 (test de Wilcoxon)] par rapport à la période de stabilisation (voir groupe composé **A** en figure 1 (A) et 1 (B)). Testé dans les mêmes conditions, le solvant n'a pas provoqué d'augmentation significative de la CV et de l'EMG-SAS. Alors qu'aucune différence significative (p>0,05 ; test de Wilcoxon) n'a été observée sur la CV (1^{ére} et 2^{éme} administrations) et sur l'EMG-SAS (1^{ére} administration), la 2^{éme} administration de solvant a induit une diminution de l'EMG-SAS [-69,59 ± 18,74% (n=8) ; p=0,035 (test de Wilcoxon)] (voir groupe solvant en figure 1 (A) et 1 (B)).

### Exemple 2 : Effet de composés de formule (I) sur la capacité vésicale in vivo chez le rat femelle anesthésié dans des conditions d'irritation vésicale induites par perfusion intra-vésicale d'acide acétique dilué

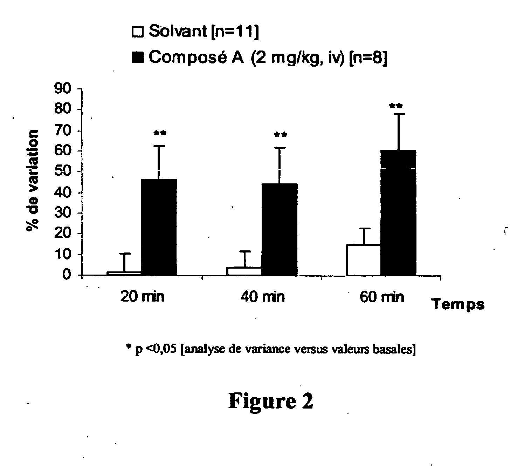

L'effet de la 4-[(2-naphtalényl)-méthoxy]-pipéridine (composé **A**) et de son solvant (NaCl 0,9%) a été étudié *in vivo* sur un modèle d'hyperactivité vésicale induite chez le rat femelle par une perfusion intra-vésicale d'acide acétique dilué (Chuang et coll., 2004). 19 rats Wistar femelles (200-280 g) ont été anesthésiés par une injection intrapéritonéale d'uréthane (1,2 g/kg). Après laparotomie, une bourse a été formée au niveau du dôme de la vessie, puis un cathéter a été introduit et ligaturé au niveau de cette bourse. Un cathéter a été implanté au niveau de la veine jugulaire pour permettre l'administration du composé **A** ou de son solvant. Une cystomanométrie a été réalisée sur les animaux préalablement placés sur une plaque chauffante, afin de maintenir leur température corporelle autour de 37°C. Un robinet à 3 voies a été positionné à l'extrémité du cathéter vésical, puis relié à une pompe permettant la perfusion de la vessie (Model «11» plus, Harvard Apparatus) et à un capteur de pression (MX 860/866 Novatrans, Medex Medical). De l'acide acétique dilué (0,3%), maintenu à température ambiante, a été perfusé dans la vessie (3 mL/h), afin d'induire des mictions successives dans des conditions d'irritation vésicale. Le profil mictionnel a été enregistré en continu grâce à un système d'acquisition de données (MacLab/8^{e}, AD Instruments) et les différents paramètres urodynamiques, dont la capacité vésicale (CV), ont été déterminés. Les valeurs basales des paramètres urodynamiques ont été déterminées après une période de stabilisation de 10 minutes. Le composé **A** (2 mg/kg) ou son solvant ont alors été injectés par voie intraveineuse sous un volume d'1 mL administré en 5 minutes à l'aide d'un pousse seringue (Model A-99, Fisher Bioblock Scientific). Les effets du composé **A** et de son solvant sur la CV ont été évalués aux temps 20, 40 et 60 minutes après l'administration et exprimés en pourcentage de variation de la valeur basale (moyenne ± erreur standard de la moyenne). Dans les deux groupes expérimentaux (composé **A** et solvant), les valeurs basales de la CV étaient identiques (p>0,05; analyse de variance) : 0,045 ± 0,005 mL et 0,042 ± 0,006 mL, respectivement, pour le solvant (n=11) et le composé **A** (n=8). Le composé **A** (2 mg/kg, i.v.) a produit une augmentation significative de la CV [46,25 ± 16,40%, 44,61 ± 17,51% et 60,34 ± 17,8%, respectivement, aux temps 20, 40 et 60 minutes suivant l'administration (p<0,05; analyse de variance)] (voir groupe composé **A** (n=8) en figure 2). Testé dans les mêmes conditions, le solvant n'a provoqué aucune modification significative de la CV quel que soit le temps de mesure considéré (p>0, 05; analyse de variance) (voir groupe solvant (n=11 ) en figure 2).

### Exemple 3 : Effet de composés de formule I in vitro sur le détrusor humain stimulé électriquement

L'effet du composé **A** et de son solvant (solution de Krebs) a été testé *in vitro* sur des strips de vessie prélevés sur 2 patients mâles (70 et 62 ans) dans le cadre d'une cystectomie radicale à cause d'un carcinome urothélial. Les strips ont été montés dans des bains à organes isolés contenant une solution de Krebs, chauffée à 37°C et oxygénée avec du carbogène (95% O₂ et 5% CO₂). Après une période de stabilisation de 60 minutes, la viabilité des strips a été testée par la réalisation d'une contraction au KCI 80 mM. Après 30 minutes de wash-out et stabilisation, les strips ont été soumis à une stimulation électrique de champ, afin de provoquer des contractions neurogéniques. Les paramètres de stimulation électrique utilisés étaient les suivants : fréquence: 30 Hz, voltage maximal, durée de l'impulsion 0,1 ms, trains de 5 s toutes les 100 s. Après stabilisation de la réponse neurogénique (environ 30 minutes après le début de la stimulation électrique), une gamme concentration-réponse cumulative au composé **A** a été réalisée entre 0,01-100 µM. Il a été montré, qu'à partir de 10 µM, le composé **A** inhibe de façon concentration-dépendante la réponse contractile des strips de détrusor à la stimulation électrique avec des pourcentages d'inhibition de 20,1 ± 4,7% à 10 µM ; 41,7 ± 5,2% à 30 µM et de 80,8 ± 6,8% à 100 µM. Testé dans les mêmes conditions, le solvant du composé **A** a un effet inhibiteur de l'ordre de 10% sur les contractions du détrusor. Ces résultats montrent que le composé **A** diminue la réponse contractile cholinergique de la vessie et pourrait ainsi être utile dans le traitement de l'incontinence urinaire d'urgence dans laquelle il existe une augmentation des contractions vésicales.

### Exemple 4 : Tests de liaison sur récepteurs sérotoninergiques humains

L'affinité du composé **A** a été évaluée *in vitro* sur 8 récepteurs sérotoninergiques humains clonés par mesure de la liaison spécifique des ligands aux récepteurs correspondants selon des méthodes adaptées de Mulheron (1994), Bryant (1996), Choi (1994), Hope (1996), Mialet (2000), Rees (1994) et Shen (1993). Les IC₅₀ et K_{¡} déterminés pour les récepteur pour lesquels le composé **A** a montré une affinité significative sont résumées dans le tableau 1.

**Tableau 1 : IC₅₀ et Ki, du composé A**

| **Récepteur (site)** | **Composé A** | |
|---|---|---|
| | **IC₅₀ (M)** | **Kᵢ(M)** |
| **5-HT_{2A} (agoniste)** | 1.0 10⁻⁶ | 6.2 10⁻⁷ |
| **5-HT_{2B} (agoniste)** | 1.0 10⁻⁶ | 9.8 10⁻⁷ |
| **5-HT_{2C} (agoniste)** | 2.5 10⁻⁷ | 2.0 10⁻⁷ |
| **5-HT₃** | 1.4 10⁻⁶ | 7.2 10⁻⁷ |
| **5-HT₄ₑ** | 4.7 10⁻⁶ | 1.6 10⁻⁶ |
| **5-HT₇** | 7.2 10⁻⁶ | 2.6 10⁻⁶ |

| | | |
|---|---|---|
| IC₅₀ : concentration entraînant la moitié de l'inhibition de la liaison spécifique du contrôle Kᵢ : constante d'inhibition (équation de Cheng Prusoff) | | |

Le composé **A** n'a pas montré d'affinité significative pour les récepteurs 5-HT_{1A} et 5-HT_{5A} (inhibition de la liaison spécifique du contrôle respectivement égale à 14% et à 1%).

### Exemple 5 : Test fonctionnel cellulaire sur le récepteur S-HT_{2C} humain cloné

Les activités agoniste et antagoniste du composé **A** ont été évaluées *in vitro* sur le récepteur 5-HT_{2C} humain exprimé dans des cellules ovariennes de hamster chinois (cellules CHO) dans une gamme de concentrations comprises entre 1 nM et 100 µM par quantification de la liaison du ligand spécifique du récepteur, le [³⁵S]GTPγS selon une méthode adaptée de Adlersberg (2000) et de Cussac (2002). Dépourvu d'activité antagoniste significative entre 1 nM et 1 µM, le composé **A** a montré une très faible activité antagoniste (18%) à 10 µM. Par ailleurs, avec une activité agoniste plafonnant entre 44% et 42% pour des concentrations comprises entre à 3 10⁻⁵ M et 10⁻⁴ M, le composé A est un agoniste partiel pour le récepteur 5-HT_{2C} pour lequel il présente une IC₅₀ de 1,5 µM.

### Exemple 6 : Test fonctionnel antagoniste sur le récepteur 5-HT₇ dans la vessie isolée de rat

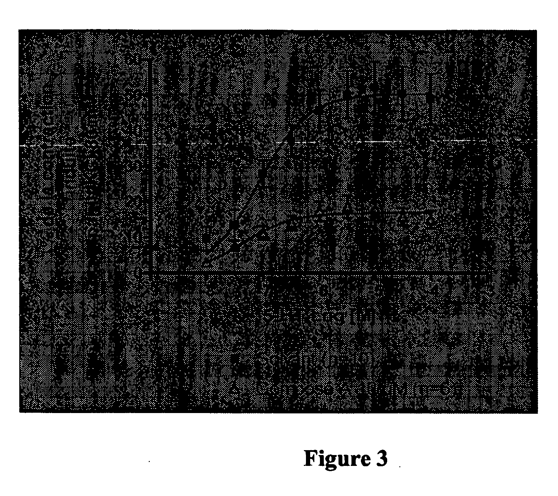

L'effet du composé **A** et de son solvant (solution de Krebs) sur la potentialisation par la sérotonine de la réponse neurogénique de la vessie isolée de rat a été étudiée *in vitro.* Des strips de détrusor prélevés chez des rats femelles Wistar (250-300 grammes) ont été montés dans des bains à organes isolés, contenant une solution de Krebs, chauffée à 37°C et oxygénée avec du carbogène (95% O₂ et 5% CO₂). Après une période de stabilisation de 60 minutes, une contraction au KCl 80 mM a été réalisée. Après 30 minutes de wash-out et stabilisation, les strips ont été soumis à une stimulation électrique de champ, afin de provoquer des contractions neurogeniques. Les paramètres de stimulation électrique utilisés étaient les suivants : fréquence: 5 Hz, voltage maximal, durée de l'impulsion 0,3 ms, trains de 10 s toutes les 60 s. Le composé **A** et son solvant sont incubés pendant 60 minutes, 30 minutes sans stimulation électrique et 30 minutes pendant la phase de stabilisation de la réponse neurogenique. Après stabilisation, une gamme concentration-réponse cumulative à la 5-HT a été réalisée entre 0,01-100 µM. Il a été montré que la 5-HT potentialise de façon concentration-dependante la réponse neurogénique du detrusor de rat, avec une réponse maximale de 52,7 ± 7,3% de la contraction de référence induite par le KCl 80 mM (voir figure 3, courbe solvant (n=10)). Après incubation en présence de 10 µM de composé A, la réponse maximale de la 5-HT n'atteint plus que 18,7 ± 3,2% de la contraction de référence induite par le KCl 80 mM (voir figure 3, courbe composé **A** (n=6)). A 10 µM le composé **A** antagonise de façon non-compétitive la réponse à la 5-HT sur la contraction neurogénique de la vessie isolée de rat ; cette action pouvant être médiée par le récepteur 5-HT₇ (Palea, 2004).

### Exemple 7 : Test fonctionnel antagoniste sur le récepteur 5-HT₄ dans la vessie humaine isolée

Des strips de détrusor humain prélevés sur des patients ayant subi une cystectomie radicale à cause d'un carcinome sont montés dans des bains à organes isolés, contenant une solution de Krebs, chauffée à 37°C et oxygénée avec du carbogène (95% O₂ et 5% CO₂). Après 60 minutes de stabilisation, une contraction au KCl 80 mM est effectuée. Après 30 minutes de wash-out et stabilisation, les strips de detrusor sont soumis à une stimulation électrique de champ, afin de provoquer des contractions neurogéniques. Les paramètres de stimulation sont les suivants: fréquence: 10 Hz, voltage maximal, durée de l'impulsion 0,1 ms, trains de 5 s toutes les 60 s. Le Composé A et son solvant (solution de Krebs) sont incubés pendant 60 minutes, 30 minutes sans stimulation électrique et 30 minutes pendant la phase de stabilisation de la réponse neurogénique. Après stabilisation, une gamme concentration-réponse cumulative à la 5-HT est réalisée entre 0,001-10 µM.

### Exemple 8 : Formulations de composés de l'invention

Cet exemple fournit différents types de formulation de composés selon l'invention, adaptées à l'utilisation thérapeutique décrite.

### Gélules dosées à 1,25 mg (base)

| | |
|---|---|
| Composé A benzoate | 1,875 mg (1,25 mg - base) |
| Cellulose microcristalline | 139,575 mg |
| Glycolate sodique d'amidon | 7,5mg |
| Stéarate de magnésium | 0,75 mg |
| Silice colloïdale | 0,3 mg |
| | pour une gélule de 150 mg |

### Gélules dosées à 2,5 mg (base)

| | |
|---|---|
| Composé **A** benzoate | 3,75 mg (2,5 mg - base) |
| Cellulose microcristalline | 279,15mg |
| Glycolate sodique d'amidon | 15mg |
| Stéarate de magnésium | 1,5 mg |
| Silice colloïdale | 0,6 mg |
| | pour une gélule de 300 mg |

### Gélules dosées à 5 mg (base)

| | |
|---|---|
| Composé **A** benzoate | 7,5 mg (5 mg - base) |
| Cellulose microcristalline | 136,5 mg |
| Glycolate sodique d'amidon | 4,5 mg |
| Stéarate de magnésium | 1,5 mg |
| | pour une gélule de 150 mg |

### Procédé de fabrication des gélules

Tamiser le composé **A**, la cellulose microcristalline, le glycolate sodique d'amidon avec un tamis approprié.

Mélanger les 3 ingrédients.

Tamiser le stéarate de magnésium et la silice colloïdale.

Lubrifier le mélange et remplir les gélules au poids théorique en utilisant une machine de remplissage automatique.

### Gélules dosées à 10 mg (base)

| | |
|---|---|
| Composé **A** benzoate | 15 mg (10 mg - base) |
| Cellulose microcristalline | 273 mg |
| Carboxyméthylamidon sodique | 9 mg |
| Stéarate de magnésium | 3 mg |
| | pour une gélule de 300 mg |

### Comprimés LP dosés à 10 mg (base)

| | |
|---|---|
| Composé **A** benzoate | 15 mg (10 mg- base) |
| Méthylhydroxypropyl cellulose | 14 mg |
| Phosphate di-calcique | 6,2 mg |
| Cellulose microcristalline sodique | 36,11 mg |
| Stéarate de magnésium | 1,05 mg |
| Silice colloïdale | 0,14 mg |
| | pour un comprimé de 72,5 mg |

### Gélules dosées de 10 à 50 mg et de 100 à 200 mg

| Composé **A** benzoate | x mg |
|---|---|
| Cellulose microcristalline | (94,25 - x) % |
| Glycolate sodique d'amidon | 3% |
| Méthylhydroxypropyl cellulose | 2% |
| Dioxyde de silicone colloïdal | 0,25% |
| Stéarate de magnésium | 0,50% |
| | pour une gélule de 200 mg (dosage de 10 à 50 mg) ou pour une gélule de 400 mg (dosage de 100 à 200 mg) |

### Suspension

| | |
|---|---|
| Composé **A** benzoate | 50 mg |
| Gomme xanthane | 4 mg |
| Cellulose microcristalline | 40 mg |
| Carboxymethylcellulose | 10 mg |
| Methyl paraben | 10 mg |
| Sucrose | 1,5 g |
| Eau purifiée q.s.p. | 5 mL |

### Bibliographie

Adlersberg M. et al., J. Neurosci. Res., 61: 674-85, 2000
Angel I. et al., Eur. J. Pharmacol., 232: 139-45, 1993
Bharucha A.E., Gastroenterology, 124 : 1672-85, 2003
Bryant H.U. et al., Life Sci., 15: 1259-68, 1996
Choi D.S. et al., FEBS Lett., 352: 393-99, 1994
Chuang Y.C. et al., J. Urol., 172: 1529-32, 2004
Cooper Z.R. et coll., Mt Sinai J.Med., 67 : 96-105, 2000
Cussac D. et al., Mol. Pharmacol., 62: 578-89, 2002
D'Agostino G. et al., J. Pharmacol. Exp. Ther., 316: 129-35, 2006**.**
Engel A.F. et coll., Int. J. Colorectal Dis., 10: 152-5, 1995
Griffiths A.N. et coll., J. Obstet. Gynaecol., 26: 442-4, 2006
Guarneri L. et al., Neurourol. Urodyn.., 15: 316-17, 1996
Haab F. et al., Neurourol. Urodyn., 25: Abst. N°64, 2006
Hope A.G. et al., Brit. J. Pharmacol.,118: 1237-45, 1996
Kamm M.A., BMJ, 316:528-32, 1998
Kapoor D.S. et coll., Int. Urogynecol. J. Pelvic Floor Dysfunct., 16: 321-8, 2005.
Lacima G. et coll., Neurourol. Urodyn., 21: 464-9, 2002
Leroi A.M. et Le Normand L., Prog. Urol., 15: 123-48, 2005
Leysen D.C., I Drugs, 2: 109-20, 1999
Macmillan A.K. et coll., Dis. Colon Rectum, 47: 1341-9, 2004
Mialet J. et al., Brit. J. Pharmacol., 129: 771-81,2000
Mulheron J.G. et al., J. Biol. Chem, 269: 12954-62, 1994
Palea S. et al., BJU, 94 : 1125-31, 2004
Perez Martinez F.C..et al., Pelv. Perineol., 1: NS102-NS103, 2006
Rao S.S., Am. J. Gastroenterol., 99: 1585-604, 2004
Rees S. et al., FEBS Lett., 355: 242-6, 1994
Roberts R.O. et coll., J. Am. Geriatr. Soc., 47: 837-41, 1999.
Scatton B. et al., Drug Dev. Research, 12: 29-40, 1988
Shen Y. et al., J. Biol. Chem., 268: 18200-04, 1993
Steers W.D. et al., Am. J. Physiol., 257: R14441-1449, 1989
Steers W.D. et al., Drug Dev. Res., 27: 361-75, 1992
Thor K.B. and Muhlhauser M.A., Am. J. Physiol., 277: R1002-R1012, 1999
Urinary incontinence: new therapeutic options, Scrip Report, Sept. 2000.
Wenzel B. et al., Neurourol. Urodyn., 25:140-147, 2006
Whitehead W.E. et coll., Gut, 45: II55-II59, 2006

## Revendications

1. Utilisation, pour la fabrication d'un médicament pour le traitement des incontinences, des troubles mictionnels associés aux dysfonctionnements du bas appareil urinaire, et/ou des troubles sphinctériens urétro-vésicaux et anaux, d'un composé de formule (I) 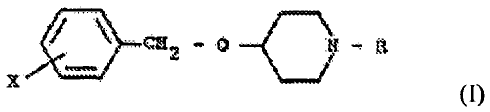 dans laquelle
R représente un atome d'hydrogène ou un radical (C1-4)alkyle, hydroxy-(C1-4)alkyle, (C1-4)alcoxycarbonyle ou benzyle, éventuellement substitué, et
X représente un ou plusieurs atomes d'hydrogène ou d'halogène ou radicaux choisis parmi (C1-4)alkyle, (C1-4)alcoxy, trifluorométhyle et méthylènedioxy, ou bien X forme avec le noyau phényle un radical naphtyle,
ainsi que ses sels et hydrates acceptables sur le plan pharmaceutique.

2. Utilisation selon la revendication 1, dans laquelle, dans la formule (I), R et X ne représentent pas simultanément des atomes d'hydrogène.

3. Utilisation selon la revendication 1 ou 2, dans laquelle, dans la formule (I), X représente un ou plusieurs atomes de chlore, ou forme avec le noyau phényle un radical naphtyle, ou représente trois radicaux méthoxy.

4. Utilisation selon l'une quelconque des revendications 1 à 3, dans laquelle, dans la formule (I), R est H.

5. Utilisation selon la revendication 1, dans laquelle le composé est la 4-[(2-naphtalényl)-méthoxy]-pipéridine ou un sel ou hydrate pharmaceutiquement acceptable de celle-ci.

6. Utilisation selon l'une quelconque des revendications précédentes, dans laquelle le composé de formule (I) est un inhibiteur de la recapture de la sérotonine, plus préférentiellement un inhibiteur sélectif de la recapture de la sérotonine.

7. Utilisation selon la revendication 6, dans laquelle le composé de formule (I) est en outre un agoniste, partiel ou non, des récepteurs 5-HT_{2C} et/ou un antagoniste des récepteurs 5-HT₃ et/ou un antagoniste des récepteurs 5-HT₃ et/ou des récepteurs 5-HT₄ et/ou récepteurs 5-HT₇.

8. Utilisation selon la revendication 6 ou 7, dans laquelle le composé de formule (I) présente une faible affinité pour les récepteurs dopaminergiques D₂, les récepteurs adrénergiques et les récepteurs muscariniques.

9. Utilisation selon l'une quelconque des revendications 1 à 8, pour le traitement des incontinences urinaires, notamment de l'incontinence d'effort, de l'incontinence d'urgence, de l'incontinence mixte, de l'incontinence par regorgement ou de l'incontinence fonctionnelle.

10. Utilisation selon l'une quelconque des revendications 1 à 8, pour le traitement des incontinences anales et fécales, et notamment des incontinences fécales vraies, des incontinences fécales fonctionnelles (IFF), des incontinences fécales passives, des incontinences fécales d'urgence et/ou du suintement fécal.

11. Utilisation selon l'une quelconque des revendications 1 à 8, pour le traitement de la pollakiurie, de l'impétuosité ou urgence urinaire, de la nocturie ou de l'énurésie.

12. Utilisation selon l'une quelconque des revendications précédentes, dans laquelle les composés sont administrés par voies orale, buccale, sublinguale, rectale, vaginale, nasale, transcutanée, parentérale, intra-vésicale, trans-urétrale ou systémique.

13. Utilisation selon l'une quelconque des revendications précédentes, dans laquelle les composés sont administrés sous forme de comprimés, pastilles, gélules, capsules molles, solutions, suspensions aqueuses ou huileuses, émulsions, solutions injectables, suppositoires, sirops, granules, poudres, patchs, gels, crèmes, pommades, sprays ou aérosols.

## Claims

1. The use, for the manufacture of a medicament for treating incontinences, micturitional disorders associated with dysfunctions of the lower urinary tract, and/or urethro-vesical and anal sphincteral disorders, of a compound of formula (I) 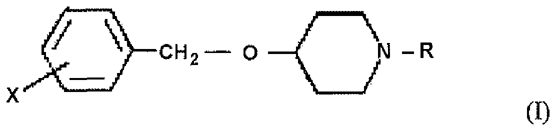 wherein
R represents a hydrogen atom or a (C₁-C₄)alkyl, hydroxyl-(C₁-C₄)alkyl, (C₁-C₄)alkoxycarbonyl or benzyl radical, optionally substituted, and
X represents one or more hydrogen or halogen atoms or radicals selected from (C₁-C₄)-alkyl, (C₁-C₄)alkoxy, trifluoromelhyl and methylenedioxy, or else X forms with the phenyl ring, a naphthyl radical,
as well as pharmaceutically acceptable salts and hydrates thereof.

2. The use according to claim 1, wherein, in formula (I), R and X do not represent hydrogen atoms simultaneously.

3. The use according to claim 1 or 2, wherein, in formula (I), X represents one or more chlorine atoms, or forms with the phenyl ring, a naphthyl radical, or represents three methoxy radicals.

4. The use according to any of claims 1 to 3, wherein, in formula (I), R is H.

5. The use according to claim 1, wherein the compound is 4-[(2-naphthalenyl)-methoxy]-piperidine or a pharmaceutically acceptable salt or hydrate thereof.

6. The use according to any of the preceding claims, wherein the compound of formula (I) is an inhibitor of serotonin recapture, more preferentially a selective inhibitor of serotonin recapture.

7. The use according to claim 6, wherein the compound of formula (I) is further an agonist, either partial or not, of 5-HT_{2C} receptors and/or an antagonist of 5-HT₃ receptors and/or an antagonist of 5-HT₃ receptors and/or of 5-HT₄ receptors and/or of 5-HT₇ receptors.

8. The use according to claim 6 or 7, wherein the compound of formula (I) has low affinity for dopaminergic receptors D₂, adrenergic receptors and muscarinic receptors.

9. The use according to any of claims 1 to 8, for treating urinary incontinences, notably stress incontinence, urgency incontinence, mixed incontinence, overflew incontinence, or functional incontinence.

10. The use according to any of claims 1 to 8, for treating anal and faecal incontinences, and notably real faecal incontinences, functional faecal incontinences (FFI), passive faecal incontinences, urgency faecal incontinences and/or of faecal seepage.

11. The use according to any of claims I to 8, for treating pollakiuria, urinary urge or urgency, nocturia or enuresis.

12. The use according to any of the preceding claims, wherein the compounds are administered through oral, buccal, sublingual, rectal, vaginal, nasal, transcutaneous, parenteral, intra-vesical, trans-urethral or systemic routes.

13. The use according to any of the preceding claims, wherein the compounds are administered as tablets, lozenges, gelatine capsules, soft capsules, solutions, aqueous or oily suspensions, emulsions, injectable solutions, suppositories, syrups, granules, powders, patches, gels, creams, ointments, sprays or aerosols.

## Patentansprüche

1. Verwendung, zur Herstellung eines Medikamentes für die Behandlung von Inkontinenzen, Miktionsstörungen, die mit Dysfunktionen des unteren Harnapparates assoziiert sind, und/oder urethrovesikalen und analen Sphinkterstörungen, einer Verbindung der Formel (I) 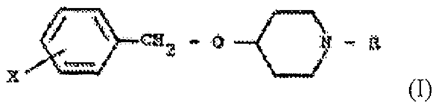 in der
R für ein Wasserstoffatom oder einen gegebenenfalls subsituierten (C₁-₄)Alkyl-, Hydroxy-(C1-4)alkyl-, (C1-4)Alkoxycarbonyl- oder Benzylrest steht, und
X für ein oder mehrere Wasserstoff oder Halogenatome oder Reste steht, die aus (C1-4)Alkyl, (C1-4)Alkoxy, Trifluormethyl und Methylendioxy ausgewählt sind, oder X mit dem Phenylring einen Naphthylrest bildet,
sowie ihre pharmazeutisch verträglichen Salze und Hydrate.

2. Verwendung gemäß Anspruch 1, wobei in der Formel (I) R und X nicht gleichzeitig Wasserstoffatome sind.

3. Verwendung gemäß Anspruch 1 oder 2, wobei in der Formel (I) X für ein oder mehrere Chloratome steht oder mit dem Phenylring einen Naphthylrest bildet oder für drei Methoxyreste steht.

4. Verwendung gemäß einem der Ansprüche 1 bis 3, wobei in der Formel (I) R H ist.

5. Verwendung gemäß Anspruch 1, wobei die Verbindung 4-[(2-Naphthalenyl)-methoxy]-piperidin oder ein pharmazeutisch verträgliches Salz oder Hydrat davon ist.

6. Verwendung gemäß einem der vorhergehenden Ansprüche, wobei die Verbindung der Formel (I) ein Hemmer der Scrotoninwiederaufnahme, vorzugsweise ein selektiver Hemmer der Serotoninwiederaufnahme, ist.

7. Verwendung gemäß Anspruch 6, wobei die Verbindung der Formel (I) außerdem ein partieller Agonist der 5-HT_{2C}-Rezeptoren sein kann und/oder ein Antagonist der 5-HT₃-Rezeptoren und/oder ein Antagonist der 5-HT₃-Rezeptoren und/oder der 5-HT₄-Rezeptoren und/oder 5-HT₇-Rezeptoren ist.

8. Verwendung gemäß Anspruch 6 oder 7, wobei die Verbindung der Formel (I) eine schwache Affinität für die dopaminergen D₂-Rezeptoren, die adrenergen Rezeptoren und die Muscarin-Rezeptoren aufweist.

9. Verwendung gemäß einem der Ansprüche 1 bis 8 für die Behandlung von Harninkontinenzen, insbesondere der Belastungsinkontinenz, der Dranginkontinenz, der Mischinkontinenz, der Überlaufinkontinenz oder der funktionellen Inkontinenz.

10. Verwendung gemäß einem der Ansprüche 1 bis 8 für die Behandlung von analen und fäkalen Inkontinenzen, und insbesondere der echten fäkalen Inkontinenzen, der funktionellen fäkalen Inkontinenzen (FFI), der passiven fäkalen Inkontinenzen, der fäkalen Dranginkontinenzen und/oder des Stuhlschmierens.

11. Verwendung gemäß einem der Ansprüche 1 bis 8 für die Behandlung der Pollakisurie, des Harndrangs oder der Urgenz, der Nykturie oder der Enuresis.

12. Verwendung gemäß einem der vorhergehenden Ansprüche, wobei die Verbindungen über den oralen, bukkalen, sublingualen, rektalen, vaginalen, nasalen, transkutanen, parenteralen, intravesikalen, transurethralen oder systemischen Weg verabreicht werden.

13. Verwendung gemäß einem der vorhergehenden Ansprüche, wobei die Verbindungen in Form von Tabletten, Pastillen, Gelkapseln, Weichkapseln, Lösungen, wässrigen oder öligen Suspensionen, Emulsionen, injizierbaren Lösungen, Suppositorien, Sirupen, Granulaten, Pulvern, Pflastern, Gelen, Cremes, Salben, Sprays oder Aerosolen verabreicht werden.
